# EUROPEAN PATENT SPECIFICATION

(11) **EP 3 379 247 B1**
(45) Date of publication and mention of the grant of the patent: **10.05.2023**
(21) Application number: 17200051.5
(22) Date of filing: 06.11.2017
(51) Int. Cl.: G01N 33/24

(54) **AN APPARATUS FOR TREATMENT OF SOIL SAMPLES**
VORRICHTUNG ZUR BEHANDLUNG VON BODENPROBEN
APPAREIL DE TRAITEMENT D'ÉCHANTILLONS DE SOL

(30) Priority: 22.03.2017 SI 201700087
(43) Date of publication of application: 26.09.2018
(73) Proprietor: Univerza v Ljubljani, 1000 Ljubljana (SI); Gozdarski institut Slovenije, 1000 Ljubljana (SI)
(72) Inventor: FERLAN, Mitja, 1352 Preserje (SI); MACEK, Irena, 1000 Ljubljana (SI); VODNIK, Dominik, 1240 Kamnik (SI)
(74) Representative: Ivancic, Bojan

(56) References cited:
- WO-A1-2007/124585
- P. ROCHETTE ET AL: "Description of a dynamic closed chamber for measuring soil respiration and its comparison with other techniques", CANADIAN JOURNAL OF SOIL SCIENCE, vol. 77, no. 2, 1 May 1997 (1997-05-01), pages 195-203, XP055482263, CA ISSN: 0008-4271, DOI: 10.4141/S96-110
- Zuazo Pablo: "Development of a fully automated soil incubation and gas sampling system for quantifying trace gas emission pulses from soils at high temporal resolution", , 1 January 2016 (2016-01-01), pages 1-183, XP055824899, DOI: 10.6094/UNIFR/12285 Retrieved from the Internet: URL:https://d-nb.info/1129080730/34 [retrieved on 2021-07-15]

## Description

The present invention refers to an apparatus for treatment of soil by supplying gas or mas mixture to a soil sample or soil samples, in order to test soil samples on low-oxygen conditions (hypoxia).

The aforementioned apparatus for treatment of soil samples has been disclosed by the publication of P. Rochette et al in the Canadian Journal of Soil Science, vol. 77, no. 2, 1 May 1997. Said publication teaches use of a dynamic closed-loop chamber for measuring soil respiration whereby air inside the chamber is thoroughly mixed by the provision of a fan. The air travels to and from the CO₂ analyser for continuous closed-loop soil respiration analysis purposes.

In addition, similar apparatus and method have been disclosed in the publication of the international patent application No. WO 2007/124585 A1. The invention disclosed therein relates generally to the field of soil gas measurement, and more specifically, to a method and apparatus for measuring soil gas surface flux.

In addition, a system for treatment of soil samples has been disclosed by the internet publication by Zuazo Pablo (Development of a fully automated soil incubation and gas sampling system for quantifying trace gas emission pulses from soils at high temporal resolution).

Known prior art teaches how to measure the gas mixture or CO₂ in the closed chamber on the surface of soil, thus, leaving the effect of natural CO₂ springs or conditions, that could happen in case of CO₂ leakage from artificial underground CO₂ storage systems, i.e. fumigating soil from below completely out of the evaluation.

It is the object of the present invention to create an apparatus for treatment of soil samples, particularly an apparatus which supplies gas or mas mixture to a soil sample or soil samples, in order to test soil samples on low-oxygen conditions.

The object as set forth is solved, according to the present invention, by an apparatus for treatment of soil samples comprising a mixing chamber and at least one assay container that are in mutual fluid communication in a closed loop. When the apparatus for treatment of soil sample or soil samples comprises plurality of assay containers, the latter are preferably interconnected in series, in parallel, or combined in series and in parallel.

The invention is further described in detail by way of non-limiting embodiment, and with a reference to the accompanying drawings, where
Fig. 1 shows a schematic view of an apparatus for treatment of soil samples according to the invention,
Fig. 2 another embodiment of the apparatus of Fig. 1,
Fig. 3 yet another embodiment of the apparatus of Fig. 1,
Fig. 4 still another embodiment of the apparatus of Fig. 1.

The invention is further described with reference to an embodiment of an apparatus for treatment of soil sample or soil samples by means of gas, such as CO₂ for example, or a gas mixture. The apparatus in accordance with independent claim 1 comprises a mixing chamber 1 having a supply extension 2 for primary gas, in particular CO₂, a discharge extension 3 for secondary gas, and a supply extension 4 for secondary gas. Said supply extension 2 for primary gas in attached inside the mixing chamber 1 to a supply pipe 5, which is separable from the supply extension 2 by means of a blocking means 6, such as by a solenoid valve for example. An impelling means 7 is arranged at the entrance of the discharge extension 3 for secondary gas that impels gas or gas mixture from the mixing chamber 1 through the discharge extension 3 for secondary gas into a duct system. The mixing chamber 1 is either from its outside or from its inside provided with a control device 8 by means of which is controlled both the blocking means 6 and the impelling means 7. Said gas, such as CO₂ for example or gas mixture is supplied to the mixing chamber 1 from a separate source, such as from a remote gas-holder. Said supply pipe 5 is led from the blocking means 6 to the immediate vicinity of the suction region of the impelling means 7.

Furthermore, at least one metering means 9, such as a pressure gauge and/or a temperature gauge and/or a gas or gas mixture concentration meter, is arranged in a mixing chamber 1, said measuring means being data-linked with said control device 8. It is provided for according to the present invention, that the supply extension 4 for the secondary gas is provided at the location before entering the mixing chamber 1 with an additional metering means 9', such as a gas or gas mixture concentration meter, which is data-linked with said control device 8.

The apparatus for treatment of soil sample or soil samples by means of gas according to the invention comprises at least one assay container 10 with a receptacle 11 of soil sample to be treated, said assay container being in fluid communication in a closed loop with said mixing chamber 1, and a gas chamber 12 arranged directly under said receptacle 11. A bottom of the receptacle 11 as well as a bottom 14 of the gas chamber 12 are perforated, thus, enabling unobstructed flow of gas and/or fluid out of and, respectively, into the soil sample to be treated. Said gas chamber 12 comprises a supply extension 15 which is in fluid communication with the discharge extension 3 for the secondary gas of the mixing chamber 1, and a discharge extension 16 which is in direct or indirect fluid communication with the supply extension 4 for the secondary gas of the mixing chamber 1. The direct fluid communication is intended in case, when the apparatus for treatment of soil sample or soil samples, in addition to the mixing chamber 1, comprises only one container 10, whereas the indirect fluid communication is intended in the case, when the apparatus for treatment of soil sample or soil samples, in addition to the mixing chamber 1 comprises a plurality of interconnected containers 10. Optionally, an additional gas and, respectively, gas mixture concentration meter which is data-linked with said control device 8, is located in the discharge extension 16 of the gas chamber 12.

Each assay container 10 is spaced from the bottom 14 arranged in a bin 17 filled with a liquid, preferably with a water, thus, preventing gas to leak through the perforated bottom 14 of the gas chamber. The height of said bin 17 is selected in a manner that the liquid contained therein covers only the perforated bottom 14 of the gas chamber 12 and does not obstruct the free flow of gas and/or gas mixture through the extensions 15, 16. In this manner, the flow of the liquid, such as water for instance, from the soil sample in the receptacle 11 through the bottom of the receptacle and further into the bin 17 and out of the latter into the surrounding is still enabled.

The apparatus for treatment of soil sample or soil samples further comprises a metering means 18 for temperature and/or moisture of the soil to be treated. Said metering means 18 is data-linked with said control device 8. In addition, each assay container 10 is provided with a heating means 19, such as a heating foil for example, which is data-linked and power-linked with said control device 8. Furthermore, each assay container 10 is protected against the external thermal influences by means of a layer 20 of a thermal insulation arranged at the side of the heating means 19 averted from the container 10. Each assay container 10 is additionally equipped with an irrigation system 21 which is preferably located in the region above the receptacle 11, and is also data-linked with said control device 8. Based on the data from the metering means 18 the control device 8 controls both the heating means 19 and the irrigation system 21 by optionally switching-on the heating means 19 in order to additionally warm the soil to be treated, and/or by switching-on the irrigation means 21 in order to additionally moisten the soil to be treated.

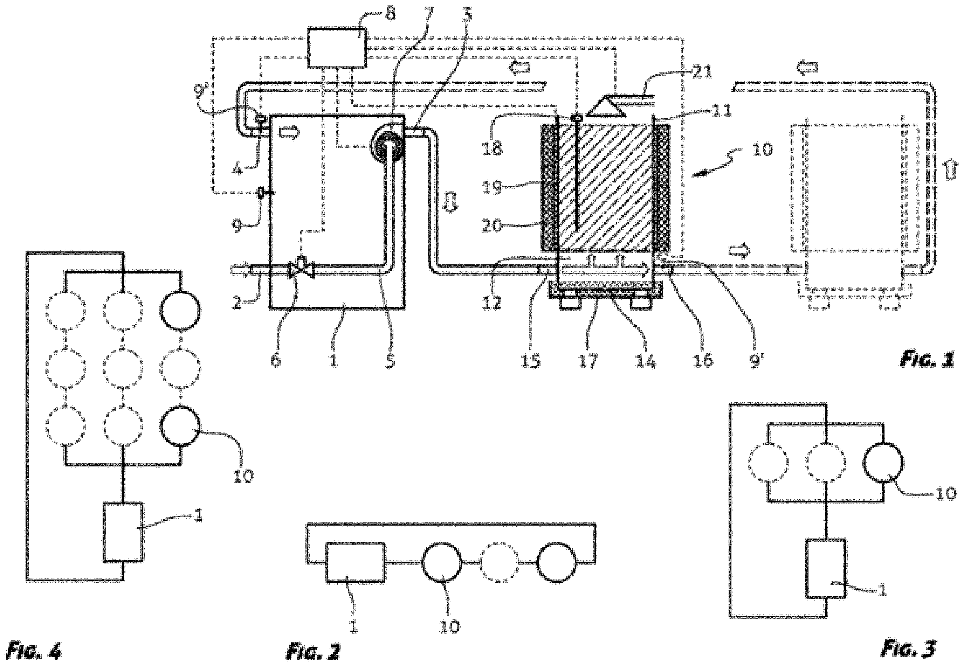

In case the apparatus for treatment of soil sample or soil samples comprises a plurality of assay containers 10, the latter are preferably interconnected in series, as shown in Fig. 2. However, an embodiment of the apparatus for treatment of soil sample or soil samples is possible where the assay containers 10 interconnected in parallel, as shown in Fig. 3, or combined in series and in parallel, as shown in Fig. 4.

Said supply extension 15 and the discharge extension 16 of each container 10 and, respectively, the gas chamber 12 thereof can be associated with the gas chamber either tangentially or centrally. Each metering mean 9, 9' and the blocking means 6 through which gas or gas mixture is supplied are linked via the control device 8 to the system, which according to predetermined programme supplies gas or gas mixture into the closed loop system. Gas or gas mixture can be fed from the gas-holder directly to the blocking means in the mixing chamber 1. When the value of gas or gas mixture, measured by metering means 9, 9', is lower than the set value, the blocking means 6 opens for a certain time, such as 10 seconds for example, and afterwards the blocking means 6 closes for a certain time, such as 10 seconds for example. After a lapse of said time, the metering is repeated and supplied, if necessary, now amount of gas or gas mixture. The impelling means 7 and the duct system that connects containers 10 and the mixing chamber 1 in the closed fluid loop enables for the gas or gas mixture, prepared in the mixing chamber 1, to flow into the gas chamber 12 of each container 10.

## Claims

1. A soil sample treatment apparatus adapted to supply a soil sample or soil samples with a gas or gas mixture comprising a mixing chamber (1) and at least one assay container (10) mutually in fluid communication in a closed loop, said mixing chamber (1) comprises a supply extension (2) for primary gas, a discharge extension (3) for secondary gas and a supply extension (4) for secondary gas, wherein said supply extension (2) for primary gas is attached inside the mixing chamber (1) to a supply pipe (5) being separable from the supply extension (2) for primary gas by means of a blocking means (6), wherein an impelling means (7) is arranged at the entrance of the discharge extension (3) for secondary gas that impels gas or gas mixture from the mixing chamber (1) through the discharge extension (3) for secondary gas into a duct system, and wherein the mixing chamber (1) is provided with a control device (8) which is adapted to control both the blocking means (6) and the impelling means (7), and at least one metering means (9) is located inside the mixing chamber (1) which is data-linked with said control device (8), wherein the supply extension (4) for secondary gas is provided at the location before entering the mixing chamber (1) with an additional metering means (9') which is data-linked with said control device (8) ***characterized in that*** a gas chamber (12) is provided directly under a receptacle (11) of said assay container (10) and wherein a bottom of the receptacle (11) as well as a bottom (14) of the gas chamber (12) is perforated enabling unobstructed flow of gas and/or fluid out of and into the soil sample to be treated, wherein each assay container (10) is spaced from the bottom (14) of the gas chamber (12) which is arranged in a bin (17) filled with a liquid preventing gas to leak through the perforated bottom (14) of the gas chamber (12).

2. An apparatus according to claim 1, ***characterized in that*** said gas chamber (12) comprises a supply extension (15) which is in fluid communication with the discharge extension (3) for the secondary gas of the mixing chamber (1), and a discharge extension (16) which is in fluid communication with the supply extension (4) for the secondary gas of the mixing chamber (1), wherein in the discharge extension (16) of the gas chamber (12) there is located a gas and, respectively, gas mixture concentration meter which is data-linked with said control device (8).

3. An apparatus according to any of preceding claims, ***characterized in that*** the liquid is preferably water and wherein the height of said bin (17) is selected in a manner that the liquid contained therein covers only the perforated bottom (14) of the gas chamber (12).

4. An apparatus according to any of the preceding claims, ***characterized in that*** each assay container (10) comprises a metering means (18) for temperature and/or moisture, a heating means (19), a thermal insulation layer (20) and an irrigation means (21).

5. An apparatus according to any of the preceding claims, ***characterized in that*** said gas or gas mixture is supplied to the mixing chamber (1) from a separate source, such as from a remote gas-holder.

6. An apparatus according to any of the preceding claims, ***characterized in that*** the metering means (9, 9') is selected as a pressure gauge and/or temperature gauge and/or a gas or gas mixture concentration meter.

7. An apparatus according to any of the preceding claims, ***characterized in that*** said supply pipe (5) extends from the blocking means (6) to the immediate vicinity of the suction region of the impelling means (7).

8. An apparatus according to any of the preceding claims, ***characterized in that*** a plurality of assay containers (10) is interconnected in series.

9. An apparatus according to any of the preceding claims, ***characterized in that*** a plurality of assay containers (10) is interconnected in parallel.

10. An apparatus according to any of the preceding claims, ***characterized in that*** a plurality of assay containers (10) is interconnected and combined in series and in parallel.

11. An apparatus according to any of the preceding claims, ***characterized in that*** said supply extension (15) and the discharge extension (16) of each gas chamber (12) are tangentially or centrally associated with said gas chamber.

## Patentansprüche

1. Bodenproben-Behandlungsvorrichtung, die eingerichtet ist, eine Bodenprobe oder Bodenproben mit einem Gas oder einem Gasgemisch zu versorgen, umfassend eine Mischkammer (1) und mindestens einen Prüfbehälter (10), die in einem geschlossenen Kreislauf miteinander in Fluidverbindung stehen, wobei die Mischkammer (1) einen Zufuhrstutzen (2) für Primärgas, einen Abfuhrstutzen (3) für Sekundärgas und einen Zufuhrstutzen (4) für Sekundärgas aufweist, wobei der Zufuhrstutzen (2) für Primärgas innerhalb der Mischkammer (1) an einem Zufuhrrohr (5) befestigt ist, das von dem Zufuhrstutzen (2) für Primärgas mittels einer Blockiereinrichtung (6) trennbar ist, wobei eine Treibeinrichtung (7) am Eingang des Abfuhrstutzens (3) für Sekundärgas angeordnet ist, die Gas oder Gasgemisch aus der Mischkammer (1) durch den Abfuhrstutzen (3) für Sekundärgas in ein Kanalsystem treibt, und wobei die Mischkammer (1) mit einer Steuervorrichtung (8) versehen ist, die eingerichtet ist, sowohl die Blockiereinrichtung (6) als auch die Treibeinrichtung (7) steuern kann, und sich mindestens eine Messeinrichtung (9) innerhalb der Mischkammer (1) befindet, die mit der Steuervorrichtung (8) datenverbunden ist, wobei der Zufuhrstutzen (4) für Sekundärgas an der Stelle vor dem Eintritt in die Mischkammer (1) mit einer zusätzlichen Messeinrichtung (9') versehen ist, die mit der Steuervorrichtung (8) datenverbunden ist,
***dadurch gekennzeichnet,* dass** eine Gaskammer (12) direkt unter einem Behältnis (11) des Prüfbehälters (10) vorgesehen ist, wobei ein Boden des Behältnisses (11) sowie ein Boden (14) der Gaskammer (12) perforiert ist, was einen ungehinderten Fluss von Gas und / oder Fluid aus der und in die zu behandelnde Bodenprobe ermöglicht, wobei jeder Prüfbehälter (10) vom Boden (14) der Gaskammer (12) beabstandet ist, die in einem mit einer Flüssigkeit gefüllten Gefäß (17) angeordnet ist, das verhindert, dass Gas durch den perforierten Boden (14) der Gaskammer (12) entweicht.

2. Vorrichtung nach Anspruch 1, ***dadurch gekennzeichnet,* dass** die Gaskammer (12) einen Zufuhrstutzen (15), der in Fluidverbindung mit dem Abfuhrstutzen (3) für das Sekundärgas der Mischkammer (1) steht, und einen Abfuhrstutzen (16), der in Fluidverbindung mit dem Zufuhrstutzen (4) für das Sekundärgas der Mischkammer (1) steht, umfasst, wobei sich in dem Abfuhrstutzen (16) der Gaskammer (12) ein Gas- beziehungsweise Gasgemischkonzentrationsmesser befindet, der mit der Steuervorrichtung (8) datenverbunden ist.

3. Vorrichtung nach einem der vorhergehenden Ansprüche, ***dadurch gekennzeichnet,* dass** die Flüssigkeit vorzugsweise Wasser ist und wobei die Höhe des Gefäßes (17) so gewählt ist, dass die darin enthaltene Flüssigkeit nur den perforierten Boden (14) der Gaskammer (12) bedeckt.

4. Vorrichtung nach einem der vorhergehenden Ansprüche, ***dadurch gekennzeichnet,* dass** jeder Prüfbehälter (10) eine Messeinrichtung (18) für Temperatur und/oder Feuchtigkeit, eine Heizeinrichtung (19), eine Wärmeisolationsschicht (20) und eine Bewässerungseinrichtung (21) umfasst.

5. Vorrichtung nach einem der vorhergehenden Ansprüche, ***dadurch gekennzeichnet,* dass** das Gas oder die Gasmischung der Mischkammer (1) von einer separaten Quelle, beispielsweise von einem entfernten Gasspeicher, zugeführt wird.

6. Vorrichtung nach einem der vorhergehenden Ansprüche, ***dadurch gekennzeichnet,* dass** die Messeinrichtung (9, 9') als Druckmesser und/oder Temperaturmesser und/oder Konzentrationsmesser für Gas oder Gasgemisch ausgewählt ist.

7. Vorrichtung nach einem der vorangehenden Ansprüche, ***dadurch gekennzeichnet,* dass** sich das Zufuhrrohr (5) von der Blockiereinrichtung (6) bis in die unmittelbare Nähe des Ansaugbereichs der Treibeinrichtung (7) erstreckt.

8. Vorrichtung nach einem der vorhergehenden Ansprüche, ***dadurch gekennzeichnet,* dass** eine Vielzahl von Prüfbehältern (10) in Reihe miteinander verbunden sind.

9. Vorrichtung nach einem der vorhergehenden Ansprüche, ***dadurch gekennzeichnet,* dass** eine Vielzahl von Prüfbehältern (10) parallel miteinander verbunden sind.

10. Vorrichtung nach einem der vorhergehenden Ansprüche, ***dadurch gekennzeichnet,* dass** eine Vielzahl von Prüfbehältern (10) in Reihe und parallel miteinander verbunden und kombiniert sind.

11. Vorrichtung nach einem der vorhergehenden Ansprüche, ***dadurch gekennzeichnet,* dass** der Zufuhrstutzen (15) und der Abfuhrstutzen (16) jeder Gaskammer (12) tangential oder mittig mit der Gaskammer verbunden sind.

## Revendications

1. - Appareil de traitement d'échantillons de sol agencé pour fournir, à un échantillon de sol ou des échantillons de sol, un gaz ou un mélange de gaz, comprenant une chambre de mélange (1) et au moins un récipient d'analyse (10) mutuellement en communication fluidique dans une boucle fermée, ladite chambre de mélange (1) comprend une extension d'alimentation (2) pour gaz principal, une extension de décharge (3) pour gaz secondaire et une extension d'alimentation (4) pour gaz secondaire, ladite extension d'alimentation (2) pour gaz principal étant fixée à l'intérieur de la chambre de mélange (1) à un tuyau d'alimentation (5) apte à être séparé de l'extension d'alimentation (2) pour gaz principal à l'aide d'un moyen de blocage (6), un moyen d'impulsion (7) étant disposé à l'entrée de l'extension de décharge (3) pour gaz secondaire qui entraîne le gaz ou le mélange de gaz à partir de la chambre de mélange (1) à travers l'extension de décharge (3) pour gaz secondaire jusque dans un système de conduit, et la chambre de mélange (1) comportant un dispositif de commande (8) qui est agencé pour commander à la fois le moyen de blocage (6) et le moyen d'impulsion (7), et au moins un moyen de mesure (9) étant situé à l'intérieur de la chambre de mélange (1), lequel est reliée par données audit dispositif de commande (8), l'extension d'alimentation (4) pour gaz secondaire comportant, à l'emplacement avant d'entrer dans la chambre de mélange (1), un moyen de mesure supplémentaire (9') qui est relié par données audit dispositif de commande (8),
**caractérisé par le fait qu'**une chambre à gaz (12) est disposée directement sous un réceptacle (11) dudit récipient d'analyse (10), et un fond du réceptacle (11) ainsi qu'un fond (14) de la chambre à gaz (12) étant perforés, permettant un écoulement, sans obstruction, de gaz et/ou de fluide hors et dans l'échantillon de sol à traiter, chaque récipient d'analyse (10) étant espacé du fond (14) de la chambre à gaz (12) qui est disposée dans un bac (17) rempli d'un liquide empêchant le gaz de fuir à travers le fond perforé (14) de la chambre à gaz (12).

2. - Appareil selon la revendication 1, **caractérisé par le fait que** ladite chambre à gaz (12) comprend une extension d'alimentation (15) qui est en communication fluidique avec l'extension de décharge (3) pour le gaz secondaire de la chambre de mélange (1), et une extension de décharge (16) qui est en communication fluidique avec l'extension d'alimentation (4) pour le gaz secondaire de la chambre de mélange (1), dans l'extension de décharge (16) de la chambre à gaz (12) se trouvant un compteur de concentration de gaz et de mélange de gaz, respectivement, qui est relié par données audit dispositif de commande (8).

3. - Appareil selon l'une quelconque des revendications précédentes, **caractérisé par le fait que** le liquide est de préférence de l'eau, et la hauteur dudit bac (17) étant choisie de telle sorte que le liquide contenu dans celui-ci couvre uniquement le fond perforé (14) de la chambre à gaz (12).

4. - Appareil selon l'une quelconque des revendications précédentes, **caractérisé par le fait que** chaque récipient d'analyse (10) comprend un moyen de mesure (18) pour température et/ou humidité, un moyen de chauffage (19), une couche d'isolation thermique (20) et un moyen d'irrigation (21).

5. - Appareil selon l'une quelconque des revendications précédentes, **caractérisé par le fait que** ledit gaz ou mélange de gaz est fourni à la chambre de mélange (1) à partir d'une source séparée, telle qu'un réservoir de gaz à distance.

6. - Appareil selon l'une quelconque des revendications précédentes, **caractérisé par le fait que** le moyen de mesure (9, 9') est choisi pour être un indicateur de pression et/ou un indicateur de température et/ou un compteur de concentration de gaz ou de mélange de gaz.

7. - Appareil selon l'une quelconque des revendications précédentes, **caractérisé par le fait que** ledit tuyau d'alimentation (5) s'étend depuis le moyen de blocage (6) jusqu'au voisinage immédiat de la région d'aspiration du moyen d'impulsion (7).

8. - Appareil selon l'une quelconque des revendications précédentes, **caractérisé par le fait qu'**une pluralité de récipients d'analyse (10) sont interconnectés en série.

9. - Appareil selon l'une quelconque des revendications précédentes, **caractérisé par le fait qu'**une pluralité de récipients d'analyse (10) sont interconnectés en parallèle.

10. - Appareil selon l'une quelconque des revendications précédentes, **caractérisé par le fait qu'**une pluralité de récipients d'analyse (10) sont interconnectés et combinés en série et en parallèle.

11. - Appareil selon l'une quelconque des revendications précédentes, **caractérisé par le fait que** ladite extension d'alimentation (15) et l'extension de décharge (16) de chaque chambre à gaz (12) sont associées à ladite chambre à gaz de manière tangentielle ou centrale.
